# EUROPEAN PATENT SPECIFICATION

(11) **EP 2 350 648 B1**
(45) Date of publication and mention of the grant of the patent: **29.03.2017**
(21) Application number: 09815297.8
(22) Date of filing: 18.09.2009
(51) Int. Cl.: G01N 33/50, C12Q 1/68

(54) **SELECTIVE PROCESSING OF BIOLOGICAL MATERIAL ON A MICROARRAY SUBSTRATE**
SELEKTIVE VERARBEITUNG BIOLOGISCHER MATERIALIEN AUF EINEM MIKROARRAY-SUBSTRAT
TRAITEMENT SÉLECTIF DE MATÉRIEL BIOLOGIQUE SUR UN SUBSTRAT À MICRORÉSEAU

(30) Priority: 16.10.2008 US 106083 P; 22.09.2008 US 99035 P
(43) Date of publication of application: 03.08.2011
(73) Proprietor: Ventana Medical Systems, Inc., Tucson, Arizona 85755 (US)
(72) Inventor: ADEY, Nils, Salt Lake City, UT 84108 (US); OLIPHANT, Arnold, Salt Lake City, UT 84119 (US); AO, Wanyuan, Salt Lake City, UT 84124 (US)
(74) Representative: Bösl, Raphael Konrad
(86) International application number: PCT/US2009/057539
(87) International publication number: WO 2010/033844

(56) References cited:
- WO-A2-03/031965
- US-A- 6 043 039
- US-A- 6 043 039
- US-A1- 2004 035 690
- US-A1- 2005 164 213
- US-A1- 2006 035 220
- US-A1- 2006 246 464
- US-A1- 2006 275 773
- US-A1- 2007 037 169
- US-A1- 2008 045 418
- US-B2- 6 806 954
- OKANO K ET AL: "Position-specific release of DNA from a chip by using photothermal denaturation", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 64, no. 1-3, 1 June 2000 (2000-06-01) , pages 88-94, XP004199291, ISSN: 0925-4005, DOI: 10.1016/S0925-4005(99)00489-X

## Description

### FIELD OF THE INVENTION

The present invention relates to the processing of biological materials on a microarray slide surface. Accordingly, the present invention involves the fields of molecular biology and chemistry.

### BACKGROUND

A microarray is a high-throughput technology that consists of an arrayed series of thousands of microscopic spots of biological material called features. A DNA microarray, for example, comprises features that contain DNA fragments of a specific DNA sequence. This can include a short section of a gene or other DNA element that is used as a probe that can hybridize to a cDNA or cRNA sample (sometimes called the target) under the proper conditions. Probe-target hybridization can be detected and quantified using fluorescence-based detection of fluorophore-labeled targets to determine relative abundance of nucleic acid sequences. In standard microarrays, probes are covalently coupled to a solid surface such as glass or silicon.
Position-specific release of DNA from a chip by using photothermal denaturation has been described in the art (Okano et al., Sensors and Actuators B64 (2000), 88-94).
Extraction methods for isolating target molecules from a sample by use of a microfluidic extraction method have been described (WO 03/031965).

### SUMMARY OF THE INVENTION

The present invention provides methods and systems for selectively eluting biological material from a distinct and/or discrete spatial location or multiple distinct and/or discrete spatial locations on a microarray slide. In one aspect, for example, a method for recovering biological material coupled to a microarray slide can include selecting a biological material to be recovered from the microarray slide, finding the biological material within a distinct or discrete spatial region on the microarray slide surface, and eluting at least a portion of the selected biological material from the distinct spatial region without eluting substantial amounts of non-selected biological material from regions of the microarray slide that are not within the distinct or discrete spatial region,
wherein eluting includes applying a denaturating buffer to the distinct or discrete spatial region,
that functions to release the portion of biological material from the microarray slide surface. In yet another specific embodiment, at least a portion of the distinct spatial region is heated to facilitate release of at least a portion of the selected biological material from the microarray surface. Numerous types of biological material are contemplated for use in the present invention, including, without limitation, DNA, cDNA, RNA, peptides, and combinations thereof.

In another aspect, a method for recovering nucleic acid material from a microarray is provided. Such a method can include selecting a nucleic acid material that has been hybridized onto a microarray slide surface in a distinct or discrete spatial region, applying a denaturing buffer to the distinct spatial region to at least partially denature the selected nucleic acid material, and flushing the microarray surface with an inert recovery buffer to recover the denatured portion of the selected nucleic acid material. In a more specific aspect, the method can further include collecting the denatured portion of the selected nucleic acid material from the inert recovery buffer. In another more specific aspect, the method can further include applying heat to the distinct spatial region to facilitate the denaturing of at least a portion of the selected nucleic acid material.

The present invention additionally provides systems for selectively eluting biological material from a distinct spatial location on a microarray slide. In one aspect, for example, a system for recovering nucleic acid material from a microarray can include a microarray scanner configured to scan a microarray surface and identify a location of a nucleic acid material to be recovered, a dispensing instrument configured to receive input from the microarray scanner and dispense an elution buffer on a discrete dispensing area of the microarray surface at the location indicated by the microarray scanner, and a recovery instrument configured to recover the elution buffer from the microarray surface.
It is also possible to receive information input from another source, such as a different array, to determine which regions to elute from. In a more specific embodiment, the system can further include a heating device configured to heat the discrete dispensing area. In another more specific embodiment the heating device is a laser.

The present invention additionally describes methods for selectively labeling biological material coupled to a microarray slide. In one aspect, such a method can include selecting a biological material to be labeled, locating the biological material within a distinct spatial region on the microarray slide surface, and labeling at least a portion of the selected biological material from the distinct spatial region without labeling substantial amounts of non-selected biological material from regions of the microarray slide that are not within the distinct spatial region. Although a variety of selective labeling techniques can be utilized, in one aspect labeling can further include applying a buffer to the distinct spatial region exclusive of regions of the microarray slide substantially outside of the distinct spatial region, and adding a label to the buffer at the distinct spatial region, such that at least a portion of the biological material within the buffer incorporates the label. It is also possible to dispense a reactive compound that can deprotect nucleic acids located at the spatial regions, or positions, of interest. These deprotected sequences can selectively react with subsequent treatments. In some aspects, this can be done using the equipment typically used to synthesize the array. In another aspect of the invention, light can be used to promote labeling and recovery of the material present at the selected location. For example, directed light can be used to deprotect reactive groups which can then react with a fluorescent or other visible tag, or a hapten such as biotin. In some aspects, this can be done using the equipment used to synthesize the array. Furthermore, the selected biological material can include any biological material capable of being labeled, including, without limitation, DNA, cDNA, RNA, peptides, and combinations thereof.

The present invention also describes methods for amplifying biological material coupled to a microarray slide. In one aspect such a method can include selecting a biological material to be amplified, locating the biological material within a distinct spatial region on the microarray slide surface, and amplifying at least a portion of the selected biological material from the distinct spatial region without amplifying substantial amounts of non-selected biological material from regions of the microarray slide that are not within the distinct spatial region. In one specific aspect, amplifying can further include applying an amplification buffer to the distinct spatial region exclusive of regions of the microarray slide substantially outside of the distinct spatial region, and amplifying at least a portion of the selected biological material within the amplification buffer. In another aspect,
light can be used to promote amplification of the material present at the selected locations. For example, light can be used to deprotect the 3' ends of nucleic acids in the selected regions and promote selected amplification. In some situations, this can be done using the equipment used to synthesize the array.

Any amplification technique capable of amplifying a biological material on the surface of a microarray slide should be considered to be within the present scope. Non-limiting examples can include isothermal cycling and thermal cycling.

There has thus been outlined, rather broadly, the more important features of the invention so that the detailed description thereof that follows may be better understood, and so that the present contribution to the art may be better appreciated. Other features of the present invention will become clearer from the following detailed description of the invention, taken with the accompanying drawings and claims, or may be learned by the practice of the invention.

### DETAILED DESCRIPTION

Before the present invention is disclosed and described, it is to be understood that terminology employed herein is used for the purpose of describing particular embodiments only and is not intended to be limiting.

It must be noted that, as used in this specification and the appended claims, the singular forms "a," "an," and "the" include plural referents unless the context clearly dictates otherwise. Thus, for example, reference to "a buffer" includes one or more of such buffers, and reference to "the chemical" includes reference to one or more of such chemicals.

### Definitions

In describing and claiming the present invention, the following terminology will be used in accordance with the definitions set forth below.

As used herein, the term "elution" refers to the act of removing a biological material from a substrate or a solution. In some aspects, such removal may be effected through the use of a liquid or fluid, such as a buffer.

As used herein, the term "distinct spatial location" refers to a distinct spatial location on a microarray slide from which biological material can be retrieved. In one aspect, the distinct spatial location can be a probe collection having a distinct border surrounding the probe collection. Such a border can include a space on the slide surface that is free of attached probe. In another aspect, the distinct spatial location can be a probe collection that is located within a larger area of deposited probe on the surface of the microarray slide, and in such a case, there may not be an area surrounding the distinct spatial location that is free of probe.

As used herein, the term "substantially" refers to the complete or nearly complete extent or degree of an action, characteristic, property, state, structure, item, or result. For example, an object that is "substantially" enclosed would mean that the object is either completely enclosed or nearly completely enclosed. The exact allowable degree of deviation from absolute completeness may in some cases depend on the specific context. However, generally speaking the nearness of completion will be so as to have the same overall result as if absolute and total completion were obtained. The use of "substantially" is equally applicable when used in a negative connotation to refer to the complete or near complete lack of an action, characteristic, property, state, structure, item, or result. For example, a composition that is "substantially free of" particles would either completely lack particles, or so nearly completely lack particles that the effect would be the same as if it completely lacked particles. In other words, a composition that is "substantially free of" an ingredient or element may still actually contain such item as long as there is no measurable effect thereof.

As used herein, the term "about" is used to provide flexibility to a numerical range endpoint by providing that a given value may be "a little above" or "a little below" the endpoint.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges such as from 1-3, from 2-4, and from 3-5, etc., as well as 1, 2, 3, 4, and 5, individually. This same principle applies to ranges reciting only one numerical value as a minimum or a maximum. Furthermore, such an interpretation should apply regardless of the breadth of the range or the characteristics being described.

### Invention

The present invention provides methods and systems for selectively eluting biological material from a distinct spatial location on a microarray slide. As has been described, in one aspect a microarray slide contains a number of specific binding or hybridization sites for assaying biological materials. In the case of a DNA microarray, for example, nucleotides having a specific sequence are clustered together at specific features, typically referred to as a probe set. A complementary nucleotide sequence can then hybridize to and thus be localized at the probe feature corresponding to the target nucleotide sequence. Accordingly, the presence of a specific nucleotide sequence in a sample can be verified due to nucleotide binding at the probe corresponding to that sequence.

Microarrays have been frequently utilized due to their high diagnostic utility. However, previous uses have often been limited to cataloguing biological materials or analyzing changes in expression levels. It has proven difficult to isolate specific sequences from the array due to the high numbers of target sequences bound to the microarray. Retrieval of a target sequence from the microarray has generally entailed denaturing all of the bound sequences from the microarray, and amplifying the target sequence of interest.

The present invention provides techniques for isolating a target sequence or a collection of target sequences from a microarray slide. Such a target sequence(s), or in other words, a biological material, is selected to be recovered from the microarray slide. The biological material can be selected as a result of the diagnostic utility of the microarray slide. For example, desired biological material can be identified based on its binding location on the microarray slide. As such, a microarray slide surface can be designed to spatially arrange probes in locations that facilitate identification and selective retrieval of biological material that binds thereto. Additionally, probes can be arranged on the microarray slide surface such that related biological material is spatially grouped together to facilitate concomitant identification and selective retrieval of the grouped biological material.

It should be noted that a collection of probes can be defined in a variety of ways, all of which should be included within the scope of the present invention. In one aspect, for example, a collection of probes could include a mixture of numerous probes that are deposited onto a microarray slide at a distinct spatial location or spot. As such, the probes may be homogenously mixed together throughout the distinct spatial location. Retrieval of biological material hybridizing to this collection of probes would include a mixture of biological material matching the mixture of probes deposited at that distinct location. In another aspect, a collection of probes can be deposited onto a microarray slide such that a distinct spatial location may include numerous single or multiple probe spots. In other words, the distinct spatial location may be made up of numerous smaller probe spots, where each probe spot contains a subset of the total collection of probes, but where the total collection of probes is represented across the collection of probe spots. In one specific aspect, each probe spot can contain a single probe sequence. In another specific aspect, each probe spot can contain a subset of probe sequences from the total collection. Biological material can be retrieved across the entire distinct spatial location, or in some cases, from a subset of the probe spots within the probe collection. It is also contemplated that the distinct spatial location can be made up of a collection of probe spots containing single probe sequences, and a collection of probes spots containing more than one probe sequence.

The selected biological material is then located in a feature, or in other words, in a distinct location on a microarray slide. At least a portion of the selected biological material is then eluted from the distinct spatial region without eluting substantial amounts of non-selected biological material from regions of the microarray slide that are not within the distinct spatial region. Such selective elution can occur in numerous ways. For example, an elution buffer can be applied to the microarray. In the context of the present invention, the elution buffer is a denaturing buffer that facilitates the denaturing of the selected biological material from the microarray. In such a case it may be beneficial to limit the application of the denaturing buffer to the distinct spatial location to avoid the denaturing of non selected biological material.

The elution buffer may be configured as a buffer that does not substantially promote denaturation of the biological material. In such cases a secondary denaturing mechanism would be applied to denature the selected biological material. For example, in one aspect an elution buffer can be applied to the distinct spatial location, and the selected biological material can be denatured via the application of a heat source to the distinct spatial location. Thus the heat generated from the heat source can be utilized to denature the biological material, which is then released from the microarray to be suspended in the elution buffer. In another aspect, an elution buffer can be applied across a larger area of the microarray, and the heat source can be applied to the distinct spatial location to denature the selected biological material. Because the elution buffer is not substantially facilitating the denaturing of biological material, primarily selected biological material should be released from the surface of the microarray slide into the elution buffer due to the localized action of the denaturing heat source. Higher heat can be utilized in this technique due to the larger volume of elution buffer available, as compared to situations where the buffer is only applied to the distinct spatial location. It should also be noted that the heat source can be applied to the distinct spatial location in the presence of a denaturing buffer to further facilitate denaturing of the selected biological material. In yet another aspect, elution can be facilitated by light. For example, if the oligonucleotides that comprise the prehybridized microarray are attached to the surface using a photo labile chemical bond, directed light can specifically cleave the oligonucleotides and the hybridized material at selected locations. In some situations, this can be done using the equipment used to synthesize the array.

Following the denaturing of the selected biological material, the microarray can be flushed with an inert recovery buffer to recover the eluted biological material. The selected biological material can then be utilized while in the recovery buffer, or such material can be further isolated from the recovery buffer using standard techniques. By repeating the application of the elution buffer, denaturing, and biological material recovery steps, separate recovery of different target materials can be accomplished from a single microarray slide.

In one specific aspect, the selected biological material can be recovered by the use of a charged surface. In the case of recovering nucleic acids, for example, the surface would have a positive charge. The charged surface can be of any geometric configuration that facilitates the recovery of the biological material. Non-limiting examples can include flat surfaces, needles, hemispheres, etc. In one aspect, a denaturing buffer can be disposed on a distinct spatial area of interest, and a positively charged surface can be introduced into the denaturing buffer to attract negatively charged biological material thereto. The charged surface can then be placed into a recovery solution, and a negative charge can be applied to the charged surface to release the biological material. One benefit of such a technique includes the ability to wash the charges surface prior to releasing the biological material to remove the denaturing buffer.

The recovered eluted biological material fragments can subsequently be used for further processes such as sequencing, hybridization, PCR, etc. In one aspect, the recovered materials could be used as input samples for sequencing. For example, the microarray can be used to isolate one or more subsets of nucleic acid fragments from the input pools, and the recovered subsets can then be used for sequencing. In some aspects, the post hybridized array may be scanned, and target nucleic acid fragments selected and individually or simultaneously collected for sequencing or other use. Location on the array can be used as part of the method of identifying target fragments for recovery. Target fragments for collection can be pre-identified in this process, or may be identified during the process. The ability to identify and collect target fragments according to the process of the present invention greatly improves the efficiency of subsequent sequencing processes.

In another aspect, the biological material can be used for *in situ* hybridization. In a more specific aspect, one *in situ* hybridization technique can include utilizing the biological material as a probe or as multiple probes for FISH (Fluorescent *in Situ* Hybridization) analysis of chromosomal regions identified by aCGH (array Comparitive Genomic Hybridization). aCGH is an array based technology used to examine genomic copy number alterations. One potential problem with aCGH is input sample heterogeneity, particularly with tumor tissue, which is often undergoing repeated genomic alterations and often mixed with non-transformed tissue. If the genomic copy number is variable in the cell population used to prepare the sample, the resulting data will represent an average of this population, and would thus reduce the sensitivity from any individual cell of interest. FISH allows examination and specific chromosomal loci quantification in individual cells.

The number of target DNA molecules in chromosomal FISH can often be very small, two per cell in normal conditions. Therefore, a significant fluorescent signal must be generated from each probe molecule in order to be seen in standard laboratory fluorescent microscopes. To compensate, nucleic acid probes (often BACs) used in FISH are typically very long (more than 100 kb) such that thousands of fluorescent dye molecules are incorporated into each probe. These BAC probes can be sheared into small DNA fragments, but the result is typically over 100 kb of the chromosomal region of interest that is targeted by fluorescently labeled probe. This is a potential problem using fragments recovered from a genome wide CGH array where the probes are located relatively sparsely along the chromosome and the labeled targets are relatively short. One example may be a 300,000 probe human CGH array hybridized with labeled targets an average of 300 base long. If one wanted to recover material to target a 100 kb region in FISH, one would recover material from just 10 probes on the microarray (300k probes/3 billion bases = 1 probe/10k bases) and just 3% of the chromosomal region of interest would be targeted (1 probe/10k bases x 300 bases ave / probe = 3%). Therefore, the potential signal would be just 3% of what one could obtain using a BAC probes, which target up to 100% of the region of interest. Therefore, it is preferable to use high density arrays and longer labeled targets in the microarray experiment. For example, if a 2.1 million probe array is hybridized using 600 bp fragments, 42% of the chromosomal region of interest would be targeted in the FISH experiment (2100k probes/3 billion bases = 1 probe/1430 bases x 600 bases ave / probe = 42%).

As has been described, the present invention provides methods to recover fragments from individual probes on a high density array. In other aspects, however, it may be desirable to create a microarray consisting of "collections" of immobilized probes, each probe in a given collection being a different sequence, and each collection of probes corresponding to a single genomic location. For example, the 3000 megabase human genome could be tested by array CGH by creating a microarray of 3000 probe collections. These collections would be spatially separated on the array such that it would be possible to denature and recover the hybridized material from every probe within a single or a small group of collections without affecting any of the neighboring collections. Denaturation and recovery could be accomplished by using a micro pipette tip that repeatedly dispensed and aspirated a tiny droplet of denaturation buffer on a given probe location. In this example, each collection would represent probes derived from sequences within a contiguous one mega base region. A collection could be made of 1000 probes spaced approximately evenly throughout the one mega base region. This process would give a one mega base resolution, in other words, allow sampling of all genomic fragments within any one mega base window for subsequent biochemical processes such as FISH.

It should also be noted that the probes immobilized in a "collection" can correspond to a variety of genomic locations. For example, probes to target sequences on biological material having similar function, or relatedness to a particular disease or condition, could be localized into a collection in order to facilitate the simultaneous recovery of functionally related biological material.

In one aspect, this technology can be particularly advantageous for FISH if the eluted labeled sample can be used directly as a FISH probe without the need for amplification. As the total quantity of eluted material is likely to be very small, the instrument should recover the eluted material in a very small volume, such as one microliter, in order to maximize the concentration in the hybridization reaction. Therefore, the tissue sample used in the FISH hybridization would need to be very small (a few millimeters or less in diameter) and the chamber volume would need to be just a few microliters. This technology is particularly well suited to microfluidic devices. Such example equipment can include, without limitation, BioMicro's 16 chamber MAUI Mixer. In another aspect, the biological material can be amplified or otherwise chemically modified subsequent to recovery from the microarray slide but prior to use.

It is additionally contemplated that all types of biological materials that can be located on a microarray slide surface can be isolated by the techniques of the present invention. Nonlimiting examples of such biological materials include DNA, cDNA, RNA, peptides, lipids, carbohydrates, etc., and combinations thereof. One of ordinary skill in the art would understand that the configurations of the microarray, the solutions and buffers, heat sources and temperatures, etc., may vary depending on the type of biological material. As such, these variations should be considered to be within the present scope.

The present invention additionally provides systems for selectively eluting biological material from a distinct spatial location on a microarray slide. In one aspect, for example, a system for recovering nucleic acid material from a microarray can include a microarray scanner configured to scan a microarray surface and identify a location of a nucleic acid material to be recovered, a dispensing instrument configured to receive input from the microarray scanner and dispense an elution buffer on a discrete dispensing area of the microarray surface at the location indicated by the microarray scanner, and a recovery instrument configured to recover the elution buffer from the microarray surface. In a more specific aspect the system can further include a heating device configured to heat the discrete dispensing area. In another more specific aspect the heating device is a laser.

As an example, the fragment elution process could be performed as follows: a hybridized and washed microarray could be scanned using a standard microarray scanner. The resulting output files would be used to guide a dispensing instrument in precise placement of a denaturing buffer on the microarray. For example, a microarray spotter such as the ArrayJet® could be used as a dispensing instrument. The denaturing buffer and protocol should efficiently elute the hybridized fragments it contacts, should not evaporate during the process, and should be recoverable without causing denaturation of fragments from unintended locations on the array. In one aspect, one possible solution is to dispense a buffer containing urea and glycerol, heat the array to cause denaturation, cooling the array to stop denaturation, and then flushing the entire array with a non denaturing recovery buffer and collect the eluted fragments.

Furthermore, the efficiency of denaturation and recovery could be significantly and continuously reduced with each round of slide washing and drying. In order to stabilize the hybridized fragments and improve specific recovery efficiency, it may be possible to develop a stabilizing wash buffer (containing special surfactants) or a microarray surface.

It should also be noted that recovery of fragments from locations on the array not subject to denaturing buffer could contaminate the eluted fragments. It can be useful to recover the elution buffer directly from the location on the array that it was dispensed. This can be accomplished by sucking the elution buffer back off the microarray using minimal recovery buffer and involving as few neighboring array locations as possible. Liquid and denatured fragments can also be recovered by blotting, or by the use of beads. This should prevent any fluid from contacting other portions of the array, which should eliminate recovery of unintended fragments.

The center to center spot distance of the features of many microarrays can be much smaller than the minimum possible size of dispensed droplets of denaturing buffer, making it difficult to recover fragments from a single spot. One possible solution is to employ a tiling array in which the spots are arranged to minimize the genomic distance between the probes in any "elution space".

In another aspect, a method is provided for selectively labeling biological material coupled to a microarray slide. In one aspect, such a method can include selecting a biological material to be labeled, locating the biological material within a distinct spatial region on the microarray slide surface, and labeling at least a portion of the selected biological material from the distinct spatial region without labeling substantial amounts of non-selected biological material from regions of the microarray slide that are not within the distinct spatial region. Although a variety of selective labeling techniques can be utilized, in one aspect labeling can further include applying a buffer to the distinct spatial region exclusive of regions of the microarray slide substantially outside of the distinct spatial region, and adding a label to the buffer at the distinct spatial region, such that at least a portion of the biological material within the buffer incorporates the label. Furthermore, the selected biological material can include any biological material capable of being labeled, including, without limitation, DNA, cDNA, RNA, peptides, and combinations thereof.

Such selectivity can include labeling just the biological material present at specific locations on the array. After the labeling step, the labeled material could be recovered from precise locations, or the labeled material along with other material could be recovered from the entire array. The technique of recovery of the labeled material along with the unlabeled (or labeled with different label) material can be employed for downstream processes such as *in situ* hybridization (ISH) if the non labeled (or labeled with different label) recovered material does not interfere with the ISH assay. An example would be a CGH array with fluorescently labeled targets. In one aspect, the spatial specific labeling could use a hapten such as biotin, a distinctive oligo or peptide sequence, or other molecules that could be distinguished in the subsequent ISH test. The advantage of using distinctive oligo or peptide sequences is multiple labeling reactions can be done simultaneously at multiple distinct locations. Following recovery from the array surface, multiple hint regions from the aCGH analysis could be tested simultaneously in the same ISH test. In some aspects, the multiple labels can also be used to subsequently affinity purify the differentially labeled and recovered fragments into different pools for downstream applications such as sequencing.

After the labeling step, various mechanisms can be utilized to release the immobilized material, such as the probes, from the microarray surface. Non-limiting mechanisms can include denaturation of hybridized or non-covalently bonded material, incorporating a reversible bond between the probe and the substrate (such as a thio bond), partial cleavage of the desired material (for example, use of a nuclease), or release en mass of all material bound to the substrate. In one specific aspect, a mechanism to release materials from specific locations on the array could utilize a photo labile bond that is broken by directed light, as this would allow for precise release of just the material of interest.

In another aspect, a method is provided for selectively amplifying biological material coupled to a microarray slide. In one aspect such a method can include selecting a biological material to be amplified, locating the biological material within a distinct spatial region on the microarray slide surface, and amplifying at least a portion of the selected biological material from the distinct spatial region without amplifying substantial amounts of non-selected biological material from regions of the microarray slide that are not within the distinct spatial region. In one specific aspect, amplifying can further include applying an amplification buffer to the distinct spatial region exclusive of regions of the microarray slide substantially outside of the distinct spatial region, and amplifying at least a portion of the selected biological material within the amplification buffer. Any amplification technique capable of amplifying a biological material on the surface of a microarray slide should be considered to be within the present scope. Non-limiting examples can include isothermal cycling and thermal cycling.

There are several potential benefits of such an approach to amplification including without limitation: 1) multiple amplifications can occur simultaneously at different locations on the array; and 2) each amplification can incorporate a different label, thus allowing all amplification products to be recovered together and each product could maintain its unique identity.

Furthermore, the following methods can be used to accomplish amplification at specific locations on a microarray: 1) the hybridized nucleic acids could be used as a template; and 2) the immobilized probes can be used as primers for a non-location specific template present in the reaction reagents. In this latter case, the spatial information can be obtained from a different source such as a different microarray.

The amplification reaction reagents including primers can be precisely applied to the desired locations using a means or mechanism such as a pipette or ink jet printer. The amplification reaction then proceeds using thermal cycling or isothermal means or methods. Various thermal and isothermal methods are known, and any such method that is suitable for the selective amplification of biological material on a microarray slide should be considered to be within the present scope.

In one aspect, the amplification can be controlled to be at discrete location by first blocking amplification at all locations on array, then precisely deblocking regions of interest. Deblocking can be precisely controlled using directed light.

## Claims

1. A method for recovering biological material coupled to a microarray slide, comprising:
selecting a biological material to be recovered from the microarray slide; finding the biological material within a distinct spatial region on the microarray slide surface; and
eluting at least a portion of the selected biological material from the distinct spatial region without eluting substantial amounts of non-selected biological material from regions of the microarray slide that are not within the distinct spatial region,
wherein eluting includes applying a denaturing buffer to the distinct spatial region that functions to release the portion of the biological material from the microarray slide.

2. The method of claim 1, wherein eluting further includes:
heating at least a portion of the distinct spatial region to facilitate release of at least a portion of the selected biological material from the microarray surface; or applying a heat source to the distinct spatial region in a presence of the denaturing buffer; and
flushing the microarray surface with a recovery buffer to recover the selected biological material.

3. The method of claim 1, wherein the selected biological material is a member selected from the group consisting of DNA, cDNA, RNA, peptides, and combinations thereof.

4. A system for recovering nucleic acid material from a microarray, comprising:
a microarray scanner configured to scan a microarray surface and identify a location of a nucleic acid material to be recovered;
a dispensing instrument configured to receive input from the microarray scanner and dispense an elution buffer on a discrete dispensing area of the microarray surface at the location indicated by the microarray scanner; and
a recovery instrument configured to recover the elution buffer from the microarray surface.

5. The system of claim 4, further comprising a heating device configured to heat the discrete dispensing area and wherein the recovery instrument is an electrically charged surface.

## Patentansprüche

1. Verfahren zum Gewinnen von biologischem Material, das an einen Mikroarrayobjektträger gekoppelt ist, umfassend:
Auswählen eines biologischen Materials, das von dem Mikroarrayobjektträger zu gewinnen ist;
Finden des biologischen Materials innerhalb einer eindeutigen räumlichen Region auf der Mikroarrayobjektträgeroberfläche; und
Eluieren von mindestens einem Teil des ausgewählten biologischen Materials von der eindeutigen räumlichen Region ohne Eluieren von wesentlichen Mengen von nicht ausgewähltem biologischem Material von Regionen des Mikroarrayobjektträgers, die nicht innerhalb der eindeutigen räumlichen Region sind,
wobei Eluieren das Auftragen eines Denaturierungspuffers auf die eindeutige räumliche Region einschließt, der funktioniert, um den Teil des biologischen Materials von dem Mikroarrayobjektträger freizusetzen.

2. Verfahren von Anspruch 1, wobei Eluieren weiterhin umfasst:
Erhitzen von mindestens einem Teil der eindeutigen räumlichen Region, um die Freisetzung von mindestens einem Teil des ausgewählten biologischen Materials von der Mikroarrayoberfläche zu ermöglichen; oder
Anwenden einer Hitzequelle auf die eindeutige räumliche Region in Anwesenheit des Denaturierungspuffers; und
Spülen der Mikroarrayoberfläche mit einem Gewinnungspuffer, um das ausgewählte biologische Material zu gewinnen.

3. Verfahren von Anspruch 1, wobei das ausgewählte biologische Material ein Mitglied, ausgewählt aus der Gruppe bestehend aus DNA, cDNA, RNA, Peptiden und Kombinationen davon, ist.

4. System zum Gewinnen von Nukleinsäurematerial von einem Mikroarray, umfassend:
einen Mikroarrayscanner, der konfiguriert ist, um eine Mikroarrayoberfläche zu durchmustern und einen Ort eines Nukleinsäurematerials, das zu gewinnen ist, zu identifizieren;
ein Verteilungsinstrument, das konfiguriert ist, um Input von dem Mikroarrayscanner zu erhalten und einen Elutionspuffer auf eine separate Verteilungsfläche der Mikroarrayoberfläche an dem Ort zu dispensieren, der von dem Mikroarrayscanner angezeigt wird; und
ein Gewinnungsinstrument, das konfiguriert ist, um den Elutionspuffer von der Mikroarrayoberfläche zu gewinnen.

5. System von Anspruch 4, weiterhin umfassend eine Heizvorrichtung, die konfiguriert ist, um die separate Verteilungsfläche zu erhitzen, und wobei das Gewinnungsinstrument eine elektrisch geladene Oberfläche ist.

## Revendications

1. Procédé pour récupérer un matériau biologique couplé à une lame à microréseau, comprenant le fait de :
sélectionner un matériau biologique à récupérer à partir de la lame à microréseau ;
détecter le matériau biologique au sein d'une zone spatiale distincte sur la surface de la lame à microréseau ; et
éluer au moins une portion du matériau biologique sélectionné à partir de la zone spatiale distincte sans éluer des quantités substantielles de matériau biologique non sélectionné à partir de zones de la lame à microréseau qui ne font pas partie de la zone spatiale distincte ;
dans lequel l'élution englobe le fait d'appliquer un tampon de dénaturation sur la zone spatiale distincte, qui a pour fonction de libérer la portion du matériau biologique à partir de la lame à microréseau.

2. Procédé selon la revendication 1, dans lequel l'élution englobe en outre le fait de :
chauffer au moins une portion de la zone spatiale distincte pour faciliter la libération d'au moins une portion du matériau biologique sélectionné à partir de la surface du microréseau ; ou
appliquer une source de chaleur sur la zone spatiale distincte en présence du tampon de dénaturation ; et
rincer la surface du microréseau avec un tampon de récupération pour récupérer le matériau biologique sélectionné.

3. Procédé selon la revendication 1, dans lequel le matériau biologique sélectionné est un membre choisi parmi le groupe constitué par de l'ADN, de l'ADNc, de l'ARN, des peptides, ainsi que leurs combinaisons.

4. Système pour récupérer un matériau d'acide nucléique à partir d'un microréseau, comprenant :
un dispositif de balayage de microréseau configuré pour balayer une surface de microréseau et identifier une localisation du matériau d'acide nucléique à récupérer ;
un instrument de distribution configuré pour recevoir une entrée à partir du dispositif de balayage du microréseau et distribuer un tampon d'élution sur une zone de distribution discrète de la surface du microréseau à l'endroit indiqué par le dispositif de balayage du microréseau ; et
un instrument de récupération configuré pour récupérer le tampon d'élution à partir de la surface du microréseau.

5. Système selon la revendication 4, comprenant en outre un dispositif de chauffage configuré pour chauffer la zone discrète de distribution et dans lequel l'instrument de récupération est une surface électriquement chargée.
